# EUROPEAN PATENT SPECIFICATION

(11) **EP 3 545 523 B1**
(45) Date of publication and mention of the grant of the patent: **18.10.2023**
(21) Application number: 17808371.3
(22) Date of filing: 16.11.2017
(51) Int. Cl.: G16H 30/40, G06V 10/764, G06V 10/82, G06V 10/98, G16H 30/20, G16H 40/63, G06T 7/00

(54) **A CLOSED-LOOP SYSTEM FOR CONTEXTUALLY-AWARE IMAGE-QUALITY COLLECTION AND FEEDBACK**
VERFAHREN ZUR KONTEXTBEWUSSTEN BILDQUALITÄTSERFASSUNG UND -RÜCKKOPPLUNG
SYSTÈME EN BOUCLE FERMÉE POUR COLLECTE ET ÉVALUATION DE QUALITÉ D'IMAGE SENSIBLE AU CONTEXTE

(30) Priority: 23.11.2016 US 201662425639 P
(43) Date of publication of application: 02.10.2019
(73) Proprietor: Koninklijke Philips N.V., 5656 AG Eindhoven (NL)
(72) Inventor: SEVENSTER, Merlijn, 5656 AE Eindhoven (NL); QIAN, Yuechen, 5656 AE Eindhoven (NL); JIA, Yugang, 5656 AE Eindhoven (NL); TAHMASEBI MARAGHOOSH, Amir, Mohammad, 5656 AE Eindhoven (NL)
(74) Representative: Philips Intellectual Property & Standards
(86) International application number: PCT/EP2017/079380
(87) International publication number: WO 2018/095792

(56) References cited:
- US-A1- 2005 251 013
- US-A1- 2007 011 024
- US-A1- 2013 083 978
- US-A1- 2014 221 832
- US-A1- 2014 369 577
- US-A1- 2015 110 348

## Description

### FIELD

The following relates to the medical arts, medical acquisition arts, medical reporting arts , and related arts.

### BACKGROUND

Medical imaging is typically performed in two phases; image acquisition and image interpretation. The acquisition is performed by technologists (or sonographers for ultrasound), who are technically trained but are not generally qualified to perform medical diagnosis based on the images. The image interpreter, oncologist, or other medical professional performs the medical diagnosis, usually at a later time (e.g. the next day or even a few days after the imaging acquisition). As a consequence, the technologist or sonographer sometimes acquire images that turn out to be diagnostically non-optimal or even non-diagnostic (i.e. the image interpreter is unable to draw diagnostic conclusions based on image acquisition deficiencies).

There is an increasing emphasis on reducing costs in medicine, including medical imaging. As a consequence, appropriateness criteria have been articulated to control the volume of medical imaging. In addition, there is increasing awareness that in the future healthcare environment (e.g. "Accountable Care Organization"), imaging departments will be expected to improve their value through high-quality image acquisition and interpretation.

As noted, image acquisition and interpretation are typically two related but temporally separated processes conducted by specialized workers. For instance, CT examinations are acquired by CT technicians and interpreted by image interpreters; cardiac echocardiogram are acquired by sonographers and interpreted by image cardiologists. (Note that in Europe cardiac echocardiograms are acquired and interpreted by image cardiologists concurrently.) Image quality can be assessed in terms of at least the following aspects and will be used in a sense covering each individually and/or together: resolution, contrast use, anatomical coverage, phase of function, motion artifact, and noise.

Even though echocardiography is the dominant modality in cardiac imaging, there is a large variability in terms of exam quality, to a point that some exams are considered non-diagnostic by expert interpreters. Low-quality image acquisition renders high-quality interpretation impossible and blocks significant value, adding to the care process and increasing costs of healthcare. In addition, low-quality image interpretation may require repeat imaging, which may require the patient to return to the hospital and delayed interpretation.

Image acquisition feedback is routinely collected at medical centers that are at the forefront of innovation. However, the feedback is reviewed periodically in the course of quality assessment programs and not used pro-actively in the image acquisition process so as to prevent low-quality images.

The following provides new and improved devices and methods which overcome the foregoing problems and others.

United States patent application publication US 2014/0369577 A1 discloses a database that stores image recipient reconstruction profiles each comprising image reconstruction parameter values. An image reconstruction module is configured to reconstruct medical imaging data to generate a reconstructed image. An image reconstruction setup module is configured to retrieve an image recipient reconstruction profile from the database for an intended image recipient associated with a set of medical imaging data and to invoke the image reconstruction module to reconstruct the set of medical imaging data using image reconstruction parameter values of the retrieved image recipient reconstruction profile to generate a reconstructed image for the intended image recipient. A feedback acquisition module is configured to acquire feedback from the intended image recipient pertaining to the reconstructed image for the intended image recipient. A profile updating module is configured to update the image recipient reconstruction profile of the intended image recipient based on the acquired feedback.

United States patent application publication US 2005/0251013 A1 discloses systems and methods for processing a medical image to automatically identify the anatomy and view (or pose) from the medical image and automatically assess the diagnostic quality of the medical image. In one aspect a method for automated decision support for medical imaging includes obtaining image data, extracting feature data from the image data, and automatically performing anatomy identification, view identification and/or determining a diagnostic quality of the image data, using the extracted feature data.

United States patent application publication US 2007/0011024 A1 discloses a business method in Health Care Management. This relates to a Global Data Center (GDC) for quality assessment and improvement in a medical imaging department by a quality assessment and improvement system (QAISys). Other healthcare areas may similarly benefit from this method. The method provides a unique way to combine computer assisted data exchange between healthcare facilities in the areas of administering, managing, and operating its Quality improvement activities. It provides feedback, instruction, and improvement ideas to enhance the skills of the personnel. National quality benchmarks for procedures and tests are established and monitored. This method is specific to the facility yet draws global national benchmarks from the database. Imaging technologists are the preferred use for this method. This provides a way to combine various existing healthcare systems with simple, new global analysis to accomplish concrete, useful and tangible improvements for the specific healthcare groups.

United States patent application publication US 2013/0083978 A1 discloses systems and methods for providing automated imaging feedback.

An example computer-implemented method of automatically providing imaging feedback includes comparing one or more of a series of first images obtained in an ongoing imaging session relating to a patient exam to one or more reference images associated with an exam type corresponding to the patient exam. The example method also includes based on the comparison, automatically generating imaging feedback. The imaging feedback comprising instructions to continue or end the imaging session.

United States patent application publication US 2014/0221832 A1 discloses the altering of values for ultrasound acquisition parameters in a manifold space. The number of parameters to be set is reduced using a manifold. Virtual parameters different than the acquisition parameters are used to alter the greater number of acquisition parameters. In a further use, optimum image settings may be obtained in an automated system by measuring image quality for feeding back to virtual parameter adjustment.

United States patent application publication US 2015/0110348 A1 relates to the analysis of optical images for diagnosing retinal problems. After being captured, the images are sent to a computing device for image quality analysis. Good quality images are sent for further processing. Poor quality images are rejected and the operator is asked to retake the image.

### BRIEF SUMMARY

In accordance with one aspect, a medical imaging apparatus includes a medical workstation with a workstation display and one or more workstation user input devices. A medical imaging device controller includes a controller display and one or more controller user input devices. The medical imaging device controller is connected to control a medical imaging device to acquire medical images. One or more electronic processors are programmed to: operate the medical workstation to provide a graphical user interface (GUI) that displays medical images stored in a radiology information system (RIS), receives entry of medical examination reports, displays an image rating user dialog, and receives, via the image rating user dialog, image quality ratings for medical images displayed at the medical workstation; operate the medical imaging device controller to perform an imaging examination session including operating the medical imaging device controller to control the medical imaging device to acquire session medical images; while performing the imaging examination session, assign quality ratings to the session medical images based on image quality ratings received via the image quality rating user dialog displayed at the medical workstation; and while performing the imaging examination session, display quality ratings assigned to the session medical images.

In accordance with another aspect, a non-transitory computer readable medium carries software to control at least one processor to perform an image acquisition method. The method includes: operating a medical workstation to provide a graphical user interface (GUI) that displays medical images stored in a medical information system (RIS), receives entry of medical examination reports, displays an image rating user dialog, and receives, via an image rating user dialog, image quality ratings for medical images displayed at the medical workstation; and performing machine learning using medical images stored in the RIS and having received image quality ratings via the image quality rating user dialog to generate a trained image quality classifier for predicting an image quality rating for an input medical image.

The invention is defined by the appended independent claims. Preferable embodiments of the invention are stated in the appended dependent claims.

One advantage resides in providing a more efficient medical workstation.

Another advantage resides in providing a medical workstation with an improved user interface.

Another advantage resides in immediately determining if the quality of acquired images is acceptable.

Another advantage resides in immediately reacquiring images of a patient if the quality of the images is substandard.

Further advantages of the present disclosure will be appreciated to those of ordinary skill in the art upon reading and understand the following detailed description. It will be appreciated that any given embodiment may achieve none, one, more, or all of the foregoing advantages and/or may achieve other advantages.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIGURE 1 shows a radiology workstation and a medical imaging device controller.
FIGURE 2 diagrammatically illustrates components of the radiology workstation and the medical imaging device
FIGURE 3 shows a flowchart showing an exemplary method of implementing the relevant prior medical study identification performed by the medical workstation of FIGURE 1.
FIGURE 4 shows a machine learning process for use with the method of FIGURE 3.

### DETAILED DESCRIPTION

The following is generally directed to a closed-loop system that provides an automated mechanism for assessing images at the time of acquisition. In this way, the technologist or sonographer is alerted if the images are not of sufficient quality and can acquire new images while the patient is still at the imaging facility.

To this end, a medical workstation is modified to provide a tool by which the image interpreter grades quality of the images being read. As the image interpreter typically carries a heavy workload, this tool should preferably make it simple for the image interpreter to provide feedback - in one embodiment the image interpreter is asked to make a selection: "Good", "Fair", or "Poor" and the images are so labeled. In this way a training dataset is efficiently collected, comprising actual medical images graded as to image quality by actual image interpreters qualified to perform such grading.

The training data are used to train a classifier (i.e. machine learning component) to receive an image (and optionally some additional context, e.g. patient characteristics, examination purpose, etc.) and output a grade, e.g. "Good", "Fair", or "Poor". The machine learning component employs deep learning comprising a neural network that receives the image directly and effectively extracts image features as outputs of the neural layers of the trained neural network. In this approach, there is no need to manually identify salient image features as this is built into the deep learning.

At the imaging laboratory, as images are acquired the trained machine learning component is applied to grade images as to "Good", "Fair", or "Poor". Any images that grade as "Poor" are preferably re-acquired, while "Fair" images may be reviewed by appropriate personnel (e.g. the imaging laboratory manager, an available image interpreter, or so forth). Advantageously, if low quality images are acquired they are identified and remedied immediately, while the patient is still at the imaging laboratory.

In some variant embodiments, the image interpreter provides more granulated image assessments, which with suitable training of multiple classifiers would allow the technologist or sonographer to receive more informational assessments, e.g. "Excessive patient motion". Less granulated assessment is also contemplated, e.g. only grades of "Good" or "Poor" (or analogous terms, e.g. "Acceptable" or "Unacceptable").

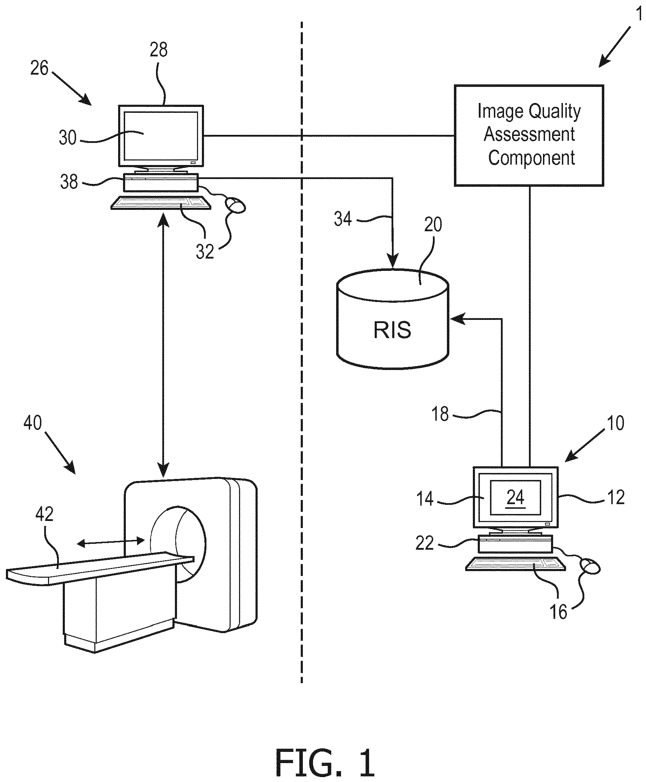

With reference to FIGURE 1, an embodiment of the disclosed rapid image quality assessment is disclosed. A medical workstation **10** may for example be implemented as a desktop computer, a "dumb" terminal connected with a network server, or any other suitable computing device to retrieve data from the server. The workstation **10** includes a computer **12** with typical components, such as at least one workstation display component **14**, at least one workstation user input component **16**, an electronic data communication link **18**, a workstation electronic database or archive **20** (e.g., an electronic medical record (EMR) database, a Picture Archiving and Communication System (PACS) database, a Radiology Information System (RIS) database, or any other suitable database). The computer **12** includes least one electronic processor **22** (e.g. a microprocessor, multi-core microprocessor, or so forth) programmed to perform medical reporting functions as disclosed herein. The at least one display **14** is configured to display one or more medical studies, and is preferably a high resolution display in order to display high resolution medical images. For example, a current study retrieved from the archive **20** can be displayed on a first display, and a previously-examined medical study, also retrieved from the archive **20**, can be displayed on a second display. In some examples, the display **14** can be a touch-sensitive display. The user input component **16** is configured to select at least one of the images. In some cases, the user input component **16** can be a mouse, a keyboard, a stylus, an aforementioned touch-sensitive display, and/or the like. In addition, the user input component **16** can be a microphone (i.e., to allow the user to dictate content to at least one of the medical reports). The communication link **18** can be a wireless or wired communication link (such as a wired or wireless Ethernet link, and/or a WiFi link), e.g. a hospital network enabling the medical workstation **10** to receive at least one image and/or at least one medical report making up a study. In addition, the archive **20** is configured to store a plurality of medical reports that include data entry fields (possibly including free-form text entry fields) by which the image interpreter enters medical findings or other observations of potential clinical significance.

The at least one processor **22** is programmed to operate the medical workstation **10** to provide a graphical user interface (GUI) **24** that displays medical images stored in the archive **20** (e.g., a RIS database), receives entry of medical examination reports, displays an image rating user dialog, and receives, via the image rating user dialog, image quality ratings for medical images displayed at the medical workstation **10.**

The medical workstation **10** provides a tool by which an image interpreter reads images of an imaging examination acquired by a technologist, sonographer, or other imaging device operator using a medical imaging device controller **26**, which may for example be implemented as a desktop computer or other suitable computing device. The medical imaging device controller **26** includes a computer **28** with typical components, such as a controller display **30**, one or more controller user input devices **32**, and a controller electronic data communication link **34** to the archive **20.** The computer includes at least one electronic processor **38** programmed to control a medical imaging device **40** to perform image acquisition functions as disclosed herein. In some examples, the display **30** can be a touch-sensitive display. In some cases, the user input device **32** can be a mouse, a keyboard, a stylus, an aforementioned touch-sensitive display, and/or the like. The communication link **34** can be a wireless or wired communication link (such as a wired or wireless Ethernet link, and/or a WiFi link), e.g. a hospital network enabling the medical imaging device controller **26** to transmit at least one image and/or at least one medical report making up a study.

The processor **38** is programmed to operate the medical imaging device controller **26** to perform an imaging examination session including operating the medical imaging device to acquire session medical images. For example, the medical imaging device controller **26** is connected to control a medical imaging device **40** (e.g., an X-ray device; a Magnetic Resonance (MR) device; a computed tomography (CT) device; an ultrasound (US) device; a positron emission tomography (PET) device; a single-photon emission computed tomography (SPECT) device; hybrids or combinations of the like (e.g., PET-CT), and the like) to acquire medical images. In some examples, the medical imaging device **40** includes a robotic subject support **42** for supporting a patient or imaging subject into the medical device. The medical imaging device controller **26** is configured to control the robotic subject support **42** to load an imaging subject into the medical imaging device **40** prior to acquiring the session medical images and to unload the imaging subject from the medical imaging device **40** after acquiring the session medical images.

In a typical arrangement, the medical imaging device controller **26** and the imaging device **40** are located in a medical laboratory, either in the same room or in adjacent rooms. For example, in the case of the imaging device **40** being an MRI, it is common practice for the controller **26** to be located in an adjacent room to limit exposure of the technician to the strong magnetic and electromagnetic fields generated by the MRI. In the case of an ultrasound device, on the other hand, the medical imaging device controller **26** and the imaging device **40** may be integrated together as a single ultrasound imaging machine including driving electronics for applying ultrasonic pulses to the ultrasound sensor array, readout electronics for reading the reflected ultrasound signals, and a built-in display, keyboard, or other user interfacing components comprising the built-in controller **26** of the ultrasound machine. These are merely illustrative examples, and other, variously integrated, embodiments of the imaging device **40** and its controller **26** are contemplated. The imaging controller **26** is connected to archive **20** to store the acquired imaging examination typically including the acquired images along with salient metadata such as image modality, reason for examination, or so forth.

While the foregoing is a typical practical arrangement in many hospitals, other arrangements of the imaging device **40** and its controller **26** may be employed. For example, in another arrangement, an ultrasound imaging device may be a portable device that is moved to the patient's hospital room to perform the ultrasound imaging examination, rather than bringing the patient to a fixed ultrasound laboratory location. The portable ultrasound device typically has its controller **26** built in, and has wireless connection to the archive **20.**

By contrast, the medical workstation **10** is typically located at a different location from the medical laboratory containing the imaging device **40** and its controller **26**, and is also not located at any patient's hospital room. Rather, the medical workstation **40** may be located in a medical department of the hospital, which is staffed by one or more image interpreters having specialized training in interpreting medical images. Typically, the technician, sonographer, or the like (who operates the medical imaging equipment **26, 40**) and the image interpreter (who uses the medical workstation **10** to perform a medical reading of the medical images) do not have significant daily interaction with each other. This creates a problematic lack of communication since the technician, sonographer, et cetera conventionally does not receive timely feedback from the image interpreter as to whether the images being acquired are suitable for the intended diagnostic task. While in principle the technician or sonographer could print out the images and consult with a image interpreter, this is usually not practical given the strict time constraints of the medical examination, the typically heavy workload borne by the image interpreter, and their locations in different parts of the hospital.

This disconnect between the technician or sonographer, on the one hand, and the image interpreter on the other hand, is addressed herein by an image quality assessment component **1**, which may be variously implemented on one or both of the processors **22**, **38** and/or on some additional processor(s) (not shown, e.g. a network-based server computer, cloud computing resource, or so forth). While the imaging examination session is being performed, the medical imaging device controller **26** is configured to transmit the images via the communication link **34** to the image quality assessment component **1**, which is configured to assign quality ratings to the session medical images based on image quality ratings received via an image quality rating user dialog to be described, which is displayed at the medical workstation **10.** The imaging device controller **30** is then configured to, while performing the imaging examination session, display quality ratings assigned to the session medical images on the display **14.** This provides the technician or sonographer with valuable feedback on image quality that can be used, in real time, i.e. during the imaging examination, to decide whether acquired images are of acceptable image quality for use in diagnostic purposes.

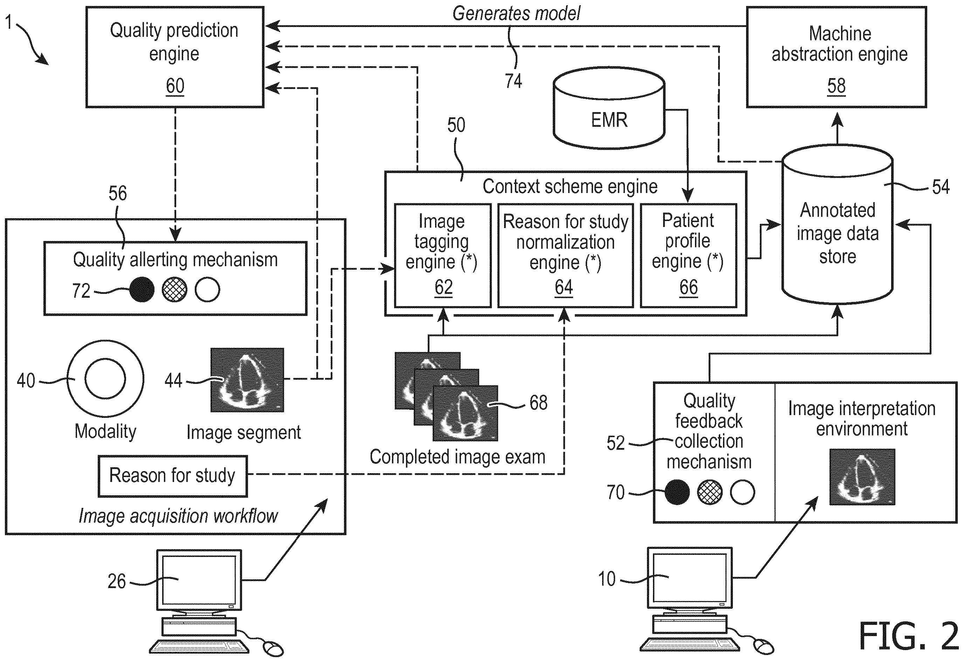

With reference to FIGURE 2, and with continuing reference to FIGURE 1, the operation of the image quality assessment component **1** and its interaction with the medical workstation **10** and the imaging device controller **30** is described in more detail. As shown in FIGURE 2, the medical workstation **10** is located in an "image interpretation environment" (e.g., a hospital medical department such as a radiology department, etc.) and the medical imaging device controller **26** is located in an "image acquisition environment" (e.g. a medical laboratory or hospital room that houses a medical imaging device, or the patient's hospital room in the case of a mobile imaging device). A technician or a sonographer (or any other suitable user) obtains at least one medical image **44** of a patient in the image acquisition environment by the medical imaging device **40** until a completed image exam is **68** obtained. The acquired images are transmitted to the medical workstation **10** in the image interpretation environment, where they are reviewed by an image interpreter (or other suitable user having specialized training in medical diagnosis of medical images). In some embodiments, the image interpreter then gives each image an image quality rating (e.g., "good", "fair", "bad", and the like). Moreover, the image quality assessment component **1** is configured to automatically assign the image quality rating (e.g., via machine-learning, as described in more detail below). If the image is given a "bad" quality rating (or, in some embodiments, a "fair" quality rating), then a notification is sent to the technician or sonographer to reacquire the image.

The machine-learning operations of the image quality assessment component **1** are described in more detail. The processor **22** of the medical workstation **10** is programmed to perform machine learning using medical images stored in the archive **20** (e.g., a RIS) and to generate a trained image quality classifier or model for predicting an image quality rating for an input medical image **44** based on the image quality ratings received via an image quality rating user dialog. The machine learning includes performing deep learning comprising training a neural network that extracts image features as outputs of one or more neural layers of the neural network. The deep learning does not operate on manually identified image features. The deep learning further uses, in addition to image features, metadata about the images as inputs to the neural network. By way of non-limiting example, the metadata about the images may include one or more of: image modality, reason for examination, and patient background stored in the archive **20.** In further examples, the machine learning is updated to update the trained image quality classifier as additional medical images are stored in the archive **20** having received image quality ratings via the image quality rating user dialog **70.**

To provide labeled training data for the machine learning, the processor **22** of the medical workstation **10** is configured (e.g. programmed) to display, on the display **14**, the image quality rating user dialog **70** while displaying medical images stored in the archive **20.** In some examples, the image quality rating user dialog **70** and the images from the archive **20** can be simultaneously displayed on a single display **14**, or individually on separate displays **14.** The image quality rating user dialog **70** is configured to receive image quality ratings for medical images stored in the archive **20** and displayed at the medical workstation display **14.** In other examples, the illustrative image quality rating user dialog **70** includes three radio selection buttons, e.g.: a green radio button indicating a selection of a "good" image quality rating; a yellow radio button indicating a selection of a "fair" image quality rating; and a red radio button indicating a selection of a "poor" image quality rating. The image interpreter can then use the selection buttons to assign ratings to the received images. This is merely an illustrative image quality rating user dialog, and other visual (e.g., graphical), auditory, or hybrid configurations are contemplated. In further non-claimed examples, the image quality rating user dialog **70** can include audible cues, in which the image interpreter can verbally state an image is "good," "fair", or "bad" and a microphone and dictation engine (items not shown) detects the verbally stated image quality rating and assigns that rating to the image.

In one embodiment, the session medical images **44** are displayed to the image interpreter at the time of acquisition. The processor **22** is then programmed to assign the image quality ratings to the session medical images **44** by inputting the session medical images to the trained image quality classifier. The image quality rating user dialog **70** provides constrained image quality ratings for user selection including at least a good image quality rating and a poor image quality rating. In this embodiment, the feedback is immediately transmitted back to the imaging device controller **26.** The display of quality ratings assigned to the session medical images includes displaying, on the controller display **28**, that any session medical image assigned the poor image quality rating should be reacquired.

A difficulty with this approach is that it requires the image interpreter to immediately review the session medical images **44** at the time of the imaging examination. This may be inconvenient for the image interpreter, or in some instances no image interpreter may be available to perform the review.

Thus, in the embodiment of FIGURE 2, to perform these feedback operations automatically, the medical imaging apparatus **1** includes an optional context scheme engine **50**, a quality feedback collection mechanism **52** for presenting the image quality rating user dialog **70** for images viewed during the normal course of the image interpreter's work, e.g. while performing readings ofpreviously acquired imaging examinations, an annotated image data store **54** that stores the images labelled (i.e. annotated) with image quality ratings from the feedback collection mechanism **52**, and a machine abstraction engine **58** that employs machine learning (e.g. deep learning of a neural network) to train an image quality classifier or quality prediction engine **60**, each of which is described in more detail below.

The optional context scheme engine **50** is programmed to transform contextually available information (e.g., modality, reason for study, patient information, and the like) into a normalized data representation. For example, the context scheme engine **50** is programmed to share normalized items of contextual information between engines. In one example, the context scheme can be a list of controlled values. For instance, if the context modality describes image modality, it can be a list of different modalities, including X-ray, CT, MR, PET, SPECT, PET-CT, and US, and it can be indicated that CT is the active modality in the given context. In another example, the context scheme can be thought of as multiple lists when the context scheme models multiple contextual aspects (e.g., modality and body part). In a further example, the context scheme can be implemented as a grid wherein some options are flagged as impossible.

In one embodiment, the context scheme engine **50** is programmed to collect information (e.g., modality, reason for study, patient information, and the like) that is available in a structured format (e.g., Digital Imaging and Communications in Medicine (DICOM) header information, metadata, patient weight, and the like). In another embodiment, the context scheme engine **50** can include an image tagging engine **62**, a reason for study normalization engine **64**, and a patient profile engine **66** that are each programmed to derive and normalize contextual information about an image, a reason for examination, and a patient's background, respectively. This normalized information can be added to the context scheme, as described in more detail below.

The image tagging engine **62** is programmed to add meta-information to the exam, such as components of the at least one image **44** (e.g., views within a cardiac echocardiogram or series in an MR study, etc.), pixel data, and the like. For example, if the image data is a cardiac echocardiogram, then the views within the exam are labelled according to their views (e.g., apical 2 chamber, apical 3 chamber, apical 4 chamber, peri-sternal long axis, etc.). In another example, certain volumetric image data can be subjected to anatomical segmentation software that automatically detects anatomies in the image data. The anatomies detected by the software may be synchronized with the ratings collected by the quality feedback collection mechanism **52**, as described in more detail below. The image tagging engine **62** is then programmed to tag each label according to its associated anatomy for each image **44** of the completed image exam **68.**

The optional reason for study normalization engine **64** is programmed to transform provided "reason for study" information into image quality requirements. As used herein, "reason for study" refers to information such as a free-text communication from the referring physician explaining symptoms, relevant chronic conditions and clinical reasoning behind the exam. Using natural language processing techniques, the reason for study normalization engine **64** is programmed to extract relevant information, and map the relevant information onto the context scheme. To do so, the reason for study normalization engine **64** is programmed to extract relevant anatomy from the reason for exam and mark any found anatomies in the context scheme. This can be implemented by making use of concept extraction methods (MetaMap or proprietary methods) that detect phrases and map them onto a concept in an ontology report, such as SNOMED CT or RadLex. Such ontologies have a relationship that interconnects their concepts and allows for hierarchical reasoning patterns. For instance, if the phrase "liver segment VI" is detected in the reason for exam, this is recognized as an anatomical location. Then, using hierarchical reasoning, the associated concept is iteratively generalized until a concept is encountered that is contained in the context scheme (e.g., "liver segment VI" → "liver").

In another embodiment, the reason for study normalization engine **64** is also programmed to recognize diseases (e.g. "hepatocarcinoma") and procedures (e.g., "prostatectomy") and leverages a pre-existing relationship modelling the relevant anatomies. Then the hierarchical reasoning can be employed, as described above, to arrive at information contained in the context scheme.

In a further embodiment, the reason for study normalization engine **64** is also programmed to map anatomical information in the reason for study onto cardiology views using ontological reasoning or basic mapping tables. For instance, a concern over the left ventricular function triggers an interest in the peri-sternal long axis, short axis, apical 4 chamber and apical 2 chamber.

The optional patient profile engine **66** is programmed to maintain a clinical disease profile of the patient. To do so, the patient profile engine **66** is programmed to collect patient information from an EMR database **68** (or, alternatively, the workstation RIS **20**) that codified using a standardized terminology, such as ICD9/ICD10 (active diagnoses) or RxNorm (active medications). The patient profile engine **66** is programmed to insert the extracted information into the context scheme.

Once generated, the context scheme generated by the context scheme engine **50** (including the information from the image tagging engine **62**, the reason for study normalization engine **64**, and the patient profile engine **66**) is transmitted to the annotated image data store **54** for storage therein. The context scheme can be, for example, formatted as metadata.

As already described, the quality feedback collection mechanism **52** is configured as a user interface device that allows an image interpreter (i.e., image interpreter) to operate the medical workstation **10** to mark low-quality images from the images **44**, or more generally to assign an image quality grade to the images. For example, selected images **44** (or, alternatively, all of the images in the completed image exam **68**) in an imaging session are transferred from the medical imaging device controller **26** to the medical workstation **10.** The received images are displayed on the display **14** of the workstation **10**, along with an image quality rating user dialog **70** of the quality feedback collection mechanism **52.** The image interpreter then uses the image quality rating user dialog **70** to assign an image quality rating to each received image **44.** For example, the image interpreter can use the workstation user input component **16** to select the image quality ratings to the images **44.** The workstation **10** then receives, via the image quality rating user dialog **70**, the image quality rating for the transferred session medical images **44.** The image quality ratings can be displayed in any suitable manner (e.g., words, colors indicated with each rating (i.e., green for "good"; yellow for "fair"; red for "poor"), and the like). The processor **22** of the workstation **10** then assigns the image quality rating received for the transferred session medical image at the medical workstation **10** to the session images **44.**

In some examples, the quality feedback collection mechanism **52** can be used by the image interpreter to mark or annotate the quality of an imaging exam or an individual series (for multi-slice medical exams) or views (for cardiac echocardiogram exams). In one embodiment, the quality feedback collection mechanism **52** enables the user to mark the image quality by assigning a image quality rating of "good"; "fair"; and "bad." In another example, the quality feedback collection mechanism **52** enables the user to mark the image quality on a Likert scale ranging from Very good, Good, Satisfactory, Borderline diagnostic, and Non-diagnostic. In a further example, a simpler quality feedback collection mechanism **52** is implemented that allows the user to only mark non-diagnostic examinations.

In yet another example, the quality feedback collection mechanism **52** allows the user to provide structured feedback that is consistent with the data representation underlying the context scheme. In a more advanced example, the quality feedback collection mechanism **52** presuggests such tags based on the outcome of the context scheme. The information can be made selectable through dropdown menus or through interactive avatars. In examples, where an image quality rating is not provided, a default quality assessment is provided.

The annotated image data store **54** is configured as non-transitory data store persisting annotated image data indexed by patient and image acquisition data. In one example, the context scheme information generated by the context scheme engine **50** is added to the annotated image data store **54.** In another example, the image quality ratings from the quality feedback collection mechanism **52** are added to the annotated image data store **54.** In another example, the completed image exam **68** can be transferred from the medical imaging device controller **26** to the annotated image data store **54.** In some embodiments, annotated image data store **54** can be configured as a cloud-based system with unique identifiers marking the source of the image content.

The quality alerting mechanism **56** is configured as a user interface configured to alert an image acquisition worker (i.e., technician or sonographer) of low-quality images. To do so, the quality alerting mechanism **56** allows the image acquisition worker to receive the image quality ratings from the image interpreter via the quality feedback collection mechanism **52.** The image quality ratings can be displayed on a image quality rating results dialog **72**, which can mirror the image quality rating user dialog **70.** For example, the images **44** and the image quality rating results dialog **72** can be displayed on the controller display **30.** The corresponding image quality ratings are then received, and then displayed on the image quality rating results dialog **72** in any suitable manner (e.g., words, colors indicated with each rating (i.e., green for "good"; yellow for "fair"; red for "poor"), and the like) that mirrors the same or substantially same manner as the image quality rating user dialog **70.** Based on the displayed image quality ratings, the image acquisition worker may then control the imaging device **40** to reacquire the desired images of the patient (e.g., the "bad" images **44**, and in some embodiments, the "fair" images).

The image quality assessment component 1 can utilize machine-learning techniques to provide the image quality ratings to the images **44.** This has the advantage of enabling immediate image quality grading without the need for an available and willing image interpreter to assign a grade directly to the current image via the quality feedback collection mechanism **52.** To enable automated image quality grading without intervention of a image interpreter to grade the current images, the image quality assessment component 1 includes the machine abstraction engine **58** and the quality prediction engine **60**, each of which is described in more detail below.

The machine abstraction engine **58** is programmed as a machine learning-enabled engine that self-leams imaging features and outputs a model that correlates such features with image quality. In some embodiments, the optional context scheme engine **50** provides further features for the correlation from the image metadata. In some examples, the machine abstraction engine **58** can be configured as a deep learning neural network that leverages multiple neural layers, with earlier neural layers in the processing sequence effectively extracting image features. Such a deep learning neural network automatically extracts image features that are encoded by neurons in the early or middle layers based on basic "atomic" image features based on ground truth annotation data. The deep learning neural network can be complemented by more complex image features that have been researched previously or developed specifically to this end.

The machine abstraction engine **58** retrieves pixel information of the images **44** from the annotated image data store **54,** as well as the image annotations from the quality feedback collection mechanism **52.** This provides a labelled training data set of images for the machine learning of the classifier **60.** The machine abstraction engine **58** is programmed to create and output an optimized mathematical model or classifier **60** that returns an image quality rating based the input image **44.** In some examples, the context scheme (including the information related to image, reason for examination, and patient background) generated by the context scheme engine **50** is also input to the machine abstraction engine **58.** The context scheme can be used by the machine abstraction engine **58** to offset image findings with contextual information to generate the output model **74.**

The generated quality prediction engine, i.e. classifier, **60** is trained by the machine learning **58** to output an image quality indicator indicating quality of the image on a pre-defined scale (e.g., "good"; "fair"; or "bad), which is then output to the quality alerting mechanism **56.** The classifier **60** thus performs the image quality grading automatically without the need for immediate availability of an image interpreter.

The image quality prediction can be augmented by other analysis. For example, a most recent prior image **44** of the patient with comparable modality and anatomy is retrieved when a low-quality rating is indicated by the classifier produced by the machine learning. In this case, logic can be applied that seeks the image segment in a prior image that matches the low-quality segment in the current image, per the quality prediction engine **60** and the context scheme. The quality indication of the prior image segment can be retrieved either from the annotated image data store **54,** or computed on the fly by the quality prediction engine **60.** If the difference in image quality is small, it may be reasoned that the image segment (e.g., echocardiogram view or anatomy in CT exam) is inherently difficult to image. This may then be included in the quality assessment and returning in a "Satisfactory" assessment.

In other examples, in which the context scheme from the context scheme engine **50** is not used as an input to the machine learning engine **58**, the output of the quality prediction engine **60** can instead be adapted based on the information from the image tagging engine **62**, the reason for study normalization engine **64**, and/or the patient profile engine **66.** For example, this information can be used to avoid flagging a low-quality concern in anatomical regions that are not necessarily relevant per the normalized reason for study. By way of illustration, a concern of noise in the upper lung area may be not crucial if the patient's presentation is suspicious for prostate cancer. Or, for instance, a reasoning rule can be applied to obese patients (ICD10 code "E66.9 - Obesity, unspecified") because certain echocardiogram views can be particularly hard to acquire for obese patients. In some examples, whenever any new image view or series is completed, the quality prediction engine **60** is applied either on the modality itself or in a PACS repository (not shown).

The quality alerting mechanism **56** is configured to receive the image ratings from the quality prediction engine **60.** If the outcome of the quality prediction engine **60** indicates that the image is non-diagnostic (e.g., graded "poor" in the previously described grading scheme), then an alert can be sent to the image acquisition worker via the quality alerting mechanism **56** of the imaging device controller **26.** In this manner, for example, the image acquisition worker can be alerted that, for example, the apical 2 chamber view is not diagnostic. In one example, a more advanced decision tree is defined that sends an appropriately formatted alert based on the output of the quality prediction engine **60.** Such a decision tree could factor in the experience level of the user and previously identified training needs. This feedback is provided automatically, during the imaging examination, so that corrective action (e.g. re-acquisition of non-diagnostic images) can be taken during the examination and the patient does not need to be recalled at a later date for a new imaging examination. Improved efficiency in the medical department is also achieved as the image interpreter no longer wastes time attempting to perform diagnosis with images of inadequate image quality.

It will be appreciated that the dotted lines shown in FIGURE 2 reflect run-time interactions between the engines. The solid lines represent interactions that serve to generate a new machine learning model.

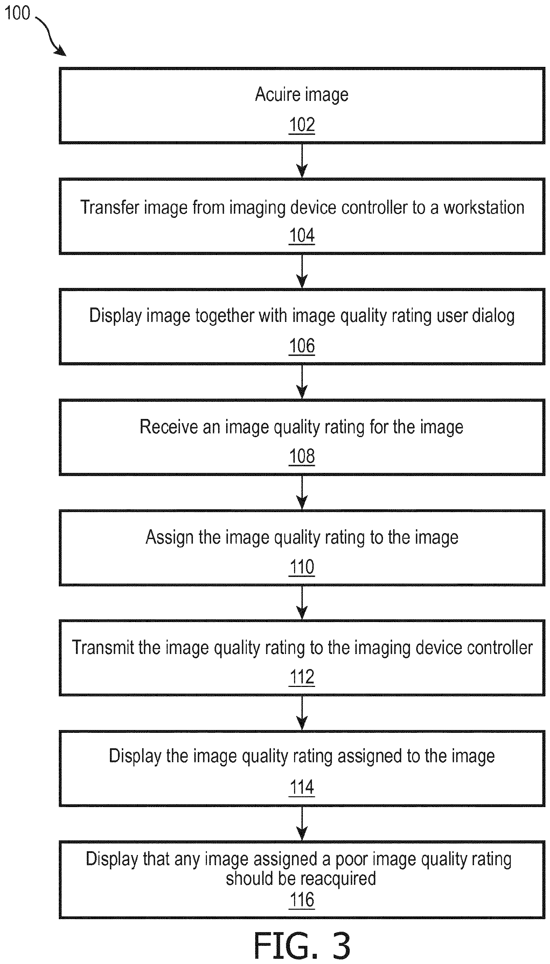

With reference to FIGURE 3, the at least one processor **22** of the workstation **10** is programmed to cause the workstation **10** to perform an image acquisition and review method **100.** The method **100** includes: acquiring session medical images **44** with a medical imaging device **40** (**102**); transferring the session medical images **44** from a medical imaging device controller **26** to a medical workstation **10** (**104**); displaying the transferred session medical image **44** at the medical workstation **10** together with the image quality rating user dialog **72** (**106**); receiving, via the image quality rating user dialog **72**, an image quality rating for the transferred session medical image **44** (**108**); assigning the session medical image **44** the image quality rating received for the transferred session medical image at the medical workstation **10** (**110**); transmit the image quality rating to the medical imaging device controller **26** (**112**); display the image quality rating assigned to the session medical image (**114**); and displaying that any session medical image assigned the poor image quality rating should be reacquired (**116**).

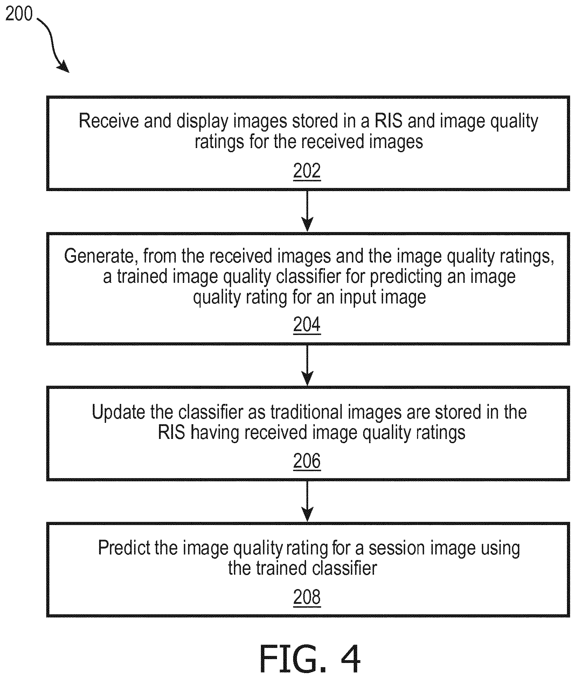

FIGURE 4 shows an alternative embodiment of the method **100.** FIGURE 4 shows steps of a machine-learning method **200** that can be performed in lieu of steps **106-110** of the method **100.** The method **200** includes: receiving and displaying medical images stored in the RIS **54** and image quality ratings via the image quality rating user dialog **70** for the received images (**202**); generate, from the received medical images and the image quality ratings, a trained image quality classifier **74** for predicting an image quality rating for an input medical image **44** (**204**); update the trained image quality classifier **74** as additional medical images are stored in the RIS **54** having received image quality ratings via the image quality rating user dialog **72** (**206**); and predicting the image quality rating for a session medical image **44** using the trained classifier **74** (**208**).

It will be appreciated that the various documents and graphical-user interface features described herein can be communicated to the various components **10, 26,** and data processing components **22, 38** via a communication network (e.g., a wireless network, a local area network, a wide area network, a personal area network, BLUETOOTH^{®}, and the like).

The various components **50, 52, 56, 58, 60, 62, 64,** of the workstation **10** can include at least one microprocessor **22, 38** programmed by firmware or software to perform the disclosed operations. In some embodiments, the microprocessor **22, 38** is integral to the various components **50, 52, 56, 58, 60, 62, 64,** so that the data processing is directly performed by the various components **50, 52, 56, 58, 60, 62, 64,** In other embodiments the microprocessor **22, 38** is separate from the various components. The data processing components **22, 38** of the workstation **10** and the medical imaging device controller **26** may also be implemented as a non-transitory storage medium storing instructions readable and executable by a microprocessor (e.g. as described above) to implement the disclosed operations. The non-transitory storage medium may, for example, comprise a read-only memory (ROM), programmable read-only memory (PROM), flash memory, or other repository of firmware for the various components **50, 52, 56, 58, 60, 62, 64,** and data processing components **22, 38.** Additionally or alternatively, the non-transitory storage medium may comprise a computer hard drive (suitable for computer-implemented embodiments), an optical disk (e.g. for installation on such a computer), a network server data storage (e.g. RAID array) from which the various component **50, 52, 56, 58, 60, 62, 64,** data processing components **22, 38** or a computer can download the device software or firmware via the Internet or another electronic data network, or so forth.

The disclosure has been described with reference to the preferred embodiments. Modifications and alterations may occur to others upon reading and understanding the preceding detailed description.

## Claims

1. A medical imaging apparatus comprising:
a medical workstation (10) including a workstation display (14) and one or more workstation user input devices (16);
a medical imaging device controller (26) including a controller display (30) and one or more controller user input devices (32), the medical imaging device controller connected to control a medical imaging device (40) to acquire medical images (44); and
one or more electronic processors (22, 38) programmed to:
operate the medical workstation to provide a graphical user interface (24) that displays medical images stored in an archive (20), receives entry of radiology examination reports, displays an image rating user dialog (70), and receives, via the image rating user dialog, image quality ratings for medical images displayed at the medical workstation;
operate the medical imaging device controller to perform an imaging examination session including operating the medical imaging device controller to control the medical imaging device to acquire session medical images;
while performing the imaging examination session, assign quality ratings to the session medical images based on image quality ratings received via the image quality rating user dialog displayed at the medical workstation; and
while performing the imaging examination session, output quality ratings assigned to the session medical images;
wherein the image quality rating user dialog (70) provides constrained image quality ratings for user selection including at least a good image quality rating and a poor image quality rating; and
wherein the display of quality ratings (72) assigned to the session medical images includes displaying that any session medical image assigned the poor image quality rating should be reacquired;
wherein the one or more electronic processors is further programmed to:
perform machine learning using medical images stored in the archive (20, 54) and having received image quality ratings via the image quality rating user dialog (70) to generate a trained image quality classifier (74) for predicting an image quality rating for an input medical image (44);
wherein quality ratings are assigned to the session medical images by inputting the session medical images to the trained image quality classifier; and wherein the machine learning includes performing deep learning comprising training a neural network (58) that extracts image features as outputs of one or more neural layers of the neural network;
wherein the deep learning further uses metadata about the images as inputs to the neural network (58), the metadata about the images including one or more of:
image modality, reason for examination, and patient background stored in the archive (20, 54);
wherein the medical imaging device is any one of the following: an X-ray device, a Magnetic Resonance device, a computer tomography device, an ultrasound device, a positron emission tomography device, a single-photon emission tomography device, and combinations thereof.

2. The medical imaging apparatus of claim 1, wherein the one or more electronic processors (22, 38) is programmed to:
display the image quality rating user dialog (70) while displaying medical images stored in the archive (20, 54); and
receive, via the image rating user dialog (70), image quality ratings for medical images stored in the archive and displayed at the medical workstation (10).

3. The medical imaging apparatus of claim 1 or 2, wherein the deep learning does not operate on manually identified image features.

4. The medical imaging apparatus of either any one of claims 1-3, wherein the one or more processors (22, 38) is further programmed to:
update the machine learning to update the trained image quality classifier (74) as additional medical images are stored in the archive (20, 54) having received image quality ratings via the image quality rating user dialog (70).

5. A non-transitory computer readable medium carrying software to control at least one processor (22, 38) of a medical imaging apparatus according to claim 1 to perform an image acquisition method, the method including
operating a medical workstation (10) to provide a graphical user interface (24) that displays medical images stored in an archive (20, 54), receives entry of radiology examination reports, displays an image rating user dialog (70), and receives, via the image rating user dialog, image quality ratings (72) for medical images displayed at the medical workstation; and
performing machine learning using medical images stored in the archive and having received image quality ratings via the image quality rating user dialog (70) to generate a trained image quality classifier (74) for predicting an image quality rating for an input medical image (44);
wherein the image quality rating user dialog (70) provides constrained image quality ratings for user selection including at least a good image quality rating and a poor image quality rating;
wherein, during a medical image reading session, the display of quality ratings assigned to the session medical images (44) includes displaying that any session medical image assigned the poor image quality rating should be reacquired;
wherein the machine learning includes performing deep learning comprising training a neural network (58) that extracts image features as outputs of one or more neural layers of the neural network;
wherein the deep learning further uses metadata about the images as inputs to the neural network (58), the metadata about the images including one or more of:
image modality, reason for examination, and patient background stored in the archive (54); and
wherein the medical imaging device is any one of the following: an X-ray device, a Magnetic Resonance device, a computer tomography device, an ultrasound device, a positron emission tomography device, a single-photon emission tomography device, and combinations thereof.

6. The non-transitory computer readable medium according to claim 5, wherein, during a medical image reading session, quality ratings are assigned to the session medical images (44) by inputting the session medical images to the trained image quality classifier (74).

7. The non-transitory computer readable medium of either one of claims 5 and 6, wherein the method further includes:
displaying the image quality rating user dialog (70) while displaying medical images stored in the archive (20, 54); and
receiving, via the image rating user dialog (70), at least one of an audio or visual image quality ratings for medical images stored in the archive (20, 54) and displayed at the medical workstation (10).

8. The non-transitory computer readable medium of claim 5, 6, or 7, wherein the deep learning does not operate on manually identified image features.

9. The non-transitory computer readable medium according to either any one of claims 5-8, wherein the method further includes:
updating the machine learning to update the trained image quality classifier (74) as additional medical images are stored in the archive (20, 54) having received image quality ratings via the image quality rating user dialog.

## Patentansprüche

1. Medizinische Bildgebungsvorrichtung, umfassend: eine medizinische Arbeitsstation (10), die eine Arbeitsstation-Anzeige (14) und eine oder mehrere Arbeitsstation-Benutzereingabevorrichtungen (16) beinhaltet;
eine Steuerung für medizinische Bildgebungsvorrichtung (26), die eine Steuerungsanzeige (30) und eine oder mehrere Benutzereingabevorrichtungen (32) beinhaltet, wobei die Steuerung für medizinische Bildgebungsvorrichtung verbunden ist, um eine medizinische Bildgebungsvorrichtung (40) zu steuern, um medizinische Bilder (44) aufzunehmen; und
einen oder mehrere elektronische Prozessoren (22, 38), die zu Folgendem programmiert sind:
Betreiben der medizinischen Arbeitsstation, um eine grafische Benutzeroberfläche (24) bereitzustellen, die in einem Archiv (20) gespeicherte medizinische Bilder anzeigt, Empfangen einer Eingabe von radiologischen Untersuchungsberichten, Anzeigen eines Bildbewertungsbenutzerdialogs (70) und Empfangen, über den Bildbewertungsbenutzerdialog, von Bildqualitätsbewertungen für medizinische Bilder, die an der medizinischen Arbeitsstation angezeigt werden;
Betreiben der Steuerung für medizinische Bildgebungsvorrichtung, um eine Bildgebungsuntersuchungssitzung auszuführen, einschließlich eines Betreibens der Steuerung für medizinische Bildgebungsvorrichtung, um die medizinische Bildgebungsvorrichtung zu steuern, um medizinische Sitzungsbilder aufzunehmen;
beim Ausführen der Bildgebungsuntersuchungssitzung, Zuweisen von Qualitätsbewertungen zu den medizinischen Sitzungsbildern basierend auf Bildqualitätsbewertungen, die über den an der medizinischen Arbeitsstation angezeigten Benutzerdialog zur Bildqualitätsbewertung empfangen werden; und
beim Ausführen der Bildgebungsuntersuchungssitzung, Ausgeben von Qualitätsbewertungen, die den medizinischen Sitzungsbildern zugewiesen werden;
wobei der Bildqualitätsbewertung-Benutzerdialog (70) eingeschränkte Bildqualitätsbewertungen für eine Benutzerauswahl bereitstellt, einschließlich mindestens einer guten Bildqualitätsbewertung und einer schlechten Bildqualitätsbewertung; und
wobei die Anzeige von Qualitätsbewertungen (72), die den medizinischen Sitzungsbildern zugewiesen sind, eine Anzeige beinhaltet, dass jedes medizinische Sitzungsbild, dem die schlechte Bildqualitätsbewertung zugewiesen wurde, erneut aufgenommen werden sollte;
wobei der eine oder die mehreren elektronischen Prozessoren ferner zu Folgendem programmiert sind:
Ausführen von maschinellem Lernen unter Verwendung von medizinischen Bildern, die in dem Archiv (20, 54) gespeichert sind und vorheriges Empfangen von Bildqualitätsbewertungen über den Bildqualitätsbewertung-Benutzerdialog (70), um einen trainierten Bildqualitätsklassifikator (74) zum Vorhersagen einer Bildqualitätsbewertung für ein eingegebenes medizinisches Bild (44) zu erzeugen;
wobei den medizinischen Sitzungsbildern Qualitätsbewertungen zugewiesen werden, indem die medizinischen Sitzungsbilder in den trainierten Bildqualitätsklassifikator eingegeben werden; und
wobei das maschinelle Lernen ein Ausführen von Deep Learning beinhaltet, umfassend ein Trainieren eines neuronalen Netzes (58), das Bildmerkmale als Ausgaben einer oder mehrerer neuronaler Schichten des neuronalen Netzes extrahiert;
wobei das Deep Learning ferner Metadaten über die Bilder als Eingaben für das neuronale Netz (58) verwendet, wobei die Metadaten über die Bilder eines oder mehrere von Folgenden beinhalten:
Bildmodalität, Grund für die Untersuchung und den in dem Archiv gespeicherten Patientenhintergrund (20, 54);
wobei die medizinische Bildgebungsvorrichtung eine der Folgenden ist: eine Röntgenvorrichtung, eine Magnetresonanzvorrichtung, eine Computertomographievorrichtung, eine Ultraschallvorrichtung, eine Positronenemissionstomographievorrichtung, eine Einzelphotonenemissionstomographievorrichtung und Kombinationen davon.

2. Medizinische Bildgebungsvorrichtung nach Anspruch 1, wobei der eine oder die mehreren elektronischen Prozessoren (22, 38) zu Folgendem programmiert sind:
Anzeigen des Bildqualitätsbewertung-Benutzerdialogs (70) beim Anzeigen der in dem Archiv (20, 54) gespeicherten medizinischen Bilder; und
Empfangen, über den Bildbewertungsbenutzerdialog (70), von Bildqualitätsbewertungen für medizinische Bilder, die in dem Archiv gespeichert sind und an der medizinischen Arbeitsstation (10) angezeigt werden.

3. Medizinische Bildgebungsvorrichtung nach Anspruch 1 oder 2, wobei das Deep Learning nicht mit manuell identifizierten Bildmerkmalen betrieben wird.

4. Medizinische Bildgebungsvorrichtung nach einem der Ansprüche 1 bis 3, wobei der eine oder die mehreren Prozessoren (22, 38) ferner zu Folgendem programmiert sind:
Aktualisieren des maschinellen Lernens, um den trainierten Bildqualitätsklassifikator (74) zu aktualisieren, wenn zusätzliche medizinische Bilder in dem Archiv (20, 54) gespeichert werden, die Bildqualitätsbewertungen über den Bildqualitätsbewertung-Benutzerdialog (70) erhalten haben.

5. Nicht-transitorisches computerlesbares Medium, das Software zum Steuern mindestens eines Prozessors (22, 38) einer medizinischen Bildgebungsvorrichtung nach Anspruch 1 enthält, um ein Bilderfassungsverfahren auszuführen, wobei das Verfahren Folgendes beinhaltet Betreiben einer medizinischen Arbeitsstation (10), um eine grafische Benutzeroberfläche (24) bereitzustellen, die in einem Archiv (20, 54) gespeicherte medizinische Bilder anzeigt, Empfangen einer Eingabe von radiologischen Untersuchungsberichten, Anzeigen eines Bildbewertungsbenutzerdialogs (70) und Empfangen, über den Bildbewertungsbenutzerdialog, von Bildqualitätsbewertungen (72) für medizinische Bilder, die an der medizinischen Arbeitsstation angezeigt werden;
Ausführen von maschinellem Lernen unter Verwendung von medizinischen Bildern, die in dem Archiv gespeichert sind und vorheriges Empfangen von Bildqualitätsbewertungen über den Bildqualitätsbewertung-Benutzerdialog (70), um einen trainierten Bildqualitätsklassifikator (74) zum Vorhersagen einer Bildqualitätsbewertung für ein eingegebenes medizinisches Bild (44) zu erzeugen;
wobei der Bildqualitätsbewertung-Benutzerdialog (70) eingeschränkte Bildqualitätsbewertungen für eine Benutzerauswahl bereitstellt, einschließlich mindestens einer guten Bildqualitätsbewertung einer schlechten Bildqualitätsbewertung;
wobei während einer medizinischen Bildlesesitzung die Anzeige von Qualitätsbewertungen, die den medizinischen Sitzungsbildern zugewiesen sind, eine Anzeige (44) beinhaltet, dass jedes medizinische Sitzungsbild, dem die schlechte Bildqualitätsbewertung zugewiesen wurde, erneut aufgenommen werden sollte;
wobei das maschinelle Lernen ein Ausführen von Deep Learning beinhaltet, umfassend ein Trainieren eines neuronalen Netzes (58), das Bildmerkmale als Ausgaben einer oder mehrerer neuronaler Schichten des neuronalen Netzes extrahiert;
wobei das Deep Learning ferner Metadaten über die Bilder als Eingaben für das neuronale Netz (58) verwendet, wobei die Metadaten über die Bilder eines oder mehrere von Bildmodalität, den Grund für die Untersuchung und den Patientenhintergrund, die in dem Archiv (54) gespeichert sind, beinhalten; und
wobei die medizinische Bildgebungsvorrichtung eine der Folgenden ist: eine Röntgenvorrichtung, eine Magnetresonanzvorrichtung, eine Computertomographievorrichtung, eine Ultraschallvorrichtung, eine Positronenemissionstomographievorrichtung, eine Einzelphotonenemissionstomographievorrichtung und Kombinationen davon.

6. Nicht-transitorisches computerlesbares Medium nach Anspruch 5, wobei während einer medizinischen Bildlesesitzung Qualitätsbewertungen den medizinischen Sitzungsbildern (44) durch Eingeben der medizinischen Sitzungsbilder in den trainierten Bildqualitätsklassifikator (74) zugewiesen werden.

7. Nicht-transitorisches computerlesbares Medium nach einem der Ansprüche 5 und 6, wobei das Verfahren ferner Folgendes beinhaltet:
Anzeigen des Bildqualitätsbewertung-Benutzerdialogs (70) beim Anzeigen der in dem Archiv (20, 54) gespeicherten medizinischen Bilder; und
Empfangen, über den Bildbewertungsbenutzerdialog (70), von mindestens einer Audio- oder visuellen Bildqualitätsbewertung für medizinische Bilder, die in dem Archiv (20, 54) gespeichert sind und an der medizinischen Arbeitsstation (10) angezeigt werden.

8. Nicht-transitorisches computerlesbares Medium nach Anspruch 5, 6 oder 7, wobei das Deep Learning nicht mit manuell identifizierten Bildmerkmalen betrieben wird.

9. Nicht-transitorisches computerlesbares Medium nach einem der Ansprüche 5-8, wobei das Verfahren ferner Folgendes beinhaltet:
Aktualisieren des maschinellen Lernens, um den trainierten Bildqualitätsklassifikator (74) zu aktualisieren, wenn zusätzliche medizinische Bilder in dem Archiv (20, 54) gespeichert werden, die Bildqualitätsbewertungen über den Bildqualitätsbewertung-Benutzerdialog erhalten haben.

## Revendications

1. Appareil d'imagerie médicale comprenant:
une station de travail médicale (10) comprenant un affichage de station de travail (14) et un ou plusieurs dispositifs d'entrée d'utilisateur de station de travail (16);
un contrôleur de dispositif d'imagerie médicale (26) comprenant un écran de contrôleur (30) et un ou plusieurs dispositifs d'entrée d'utilisateur de contrôleur (32), le contrôleur de dispositif d'imagerie médicale étant connecté pour contrôler un dispositif d'imagerie médicale (40) afin d'acquérir des images médicales (44); et
un ou plusieurs processeurs électroniques (22, 38) programmés pour:
faire fonctionner le poste de travail médical pour fournir une interface utilisateur graphique (24) qui affiche des images médicales stockées dans une archive (20), reçoit l'entrée de rapports d'examen radiologique, affiche une boîte de dialogue utilisateur d'évaluation d'image (70) et reçoit, via l'utilisateur d'évaluation d'image boîte de dialogue, cotes de qualité d'image pour les images médicales affichées au poste de travail médical;
faire fonctionner le contrôleur de dispositif d'imagerie médicale pour effectuer une session d'examen d'imagerie, y compris faire fonctionner le contrôleur de dispositif d'imagerie médicale pour commander le dispositif d'imagerie médicale afin d'acquérir des images médicales de session;
lors de l'exécution de la session d'examen d'imagerie, attribuer des évaluations de qualité aux images médicales de session sur la base d'évaluations de qualité d'image reçues via la boîte de dialogue utilisateur d'évaluation de qualité d'image affichée sur le poste de travail médical; et
lors de l'exécution de la session d'examen d'imagerie, des notes de qualité de sortie attribuées aux images médicales de la session;
dans lequel la boîte de dialogue utilisateur d'évaluation de la qualité d'image (70) fournit des évaluations de qualité d'image contraintes pour la sélection de l'utilisateur comprenant au moins une évaluation de bonne qualité d'image et une évaluation de qualité d'image médiocre; et
dans lequel l'affichage d'évaluations de qualité (72) attribuées aux images médicales de session comprend l'affichage que toute image médicale de session attribuée à l'évaluation de mauvaise qualité d'image doit être réacquise;
dans lequel le ou les processeurs électroniques sont en outre programmés pour:
effectuer un apprentissage automatique à l'aide d'images médicales stockées dans l'archive (20, 54) et ayant reçu des évaluations de qualité d'image via le dialogue utilisateur d'évaluation de qualité d'image (70) pour générer un classificateur de qualité d'image formé (74) pour prédire une évaluation de qualité d'image pour une entrée image médicale (44);
dans lequel des évaluations de qualité sont attribuées aux images médicales de session en entrant les images médicales de session dans le classificateur de qualité d'image formé; et dans lequel l'apprentissage automatique comprend l'exécution d'un apprentissage approfondi comprenant l'apprentissage d'un réseau neuronal (58) qui extrait des caractéristiques d'image en tant que sorties d'une ou plusieurs couches neuronales du réseau neuronal;
dans lequel l'apprentissage en profondeur utilise en outre des métadonnées sur les images en tant qu'entrées dans le réseau neuronal (58), les métadonnées sur les images comprenant un ou plusieurs parmi:
modalité d'image, raison de l'examen et antécédents du patient stockés dans les archives (20, 54);
dans lequel le dispositif d'imagerie médicale est l'un quelconque des éléments suivants: un appareil à rayons X, un appareil à résonance magnétique, un appareil de tomographie par ordinateur, un appareil à ultrasons, un appareil de tomographie par émission de positrons, un appareil de tomographie par émission à photon unique, et des combinaisons de ceux-ci.

2. Appareil d'imagerie médicale selon la revendication 1, dans lequel le ou les processeurs électroniques (22, 38) sont programmés pour:
afficher la boîte de dialogue utilisateur d'évaluation de la qualité d'image (70) tout en affichant des images médicales stockées dans l'archive (20, 54); et
recevoir, via la boîte de dialogue utilisateur d'évaluation d'image (70), des évaluations de qualité d'image pour des images médicales stockées dans l'archive et affichées sur le poste de travail médical (10).

3. Appareil d'imagerie médicale selon la revendication 1 ou 2, dans lequel l'apprentissage en profondeur n'opère pas sur des caractéristiques d'image identifiées manuellement.

4. Appareil d'imagerie médicale selon l'une quelconque des revendications 1 à 3, dans lequel le ou les processeurs (22, 38) sont en outre programmés pour:
mettre à jour l'apprentissage automatique pour mettre à jour le classificateur de qualité d'image formé (74) lorsque des images médicales supplémentaires sont stockées dans l'archive (20, 54) ayant reçu des évaluations de qualité d'image via le dialogue utilisateur d'évaluation de qualité d'image (70).

5. Support lisible par ordinateur non transitoire portant un logiciel pour commander au moins un processeur (22, 38) d'un appareil d'imagerie médicale selon la revendication 1 pour exécuter un procédé d'acquisition d'image, le procédé comprenant
actionner une station de travail médicale (10) pour fournir une interface utilisateur graphique (24) qui affiche des images médicales stockées dans une archive (20, 54), reçoit l'entrée de rapports d'examen radiologique, affiche une boîte de dialogue utilisateur d'évaluation d'image (70) et reçoit, via la boîte de dialogue utilisateur d'évaluation d'image, des évaluations de qualité d'image (72) pour des images médicales affichées sur le poste de travail médical; et effectuer un apprentissage automatique à l'aide d'images médicales stockées dans l'archive et ayant reçu des évaluations de qualité d'image via le dialogue utilisateur d'évaluation de qualité d'image (70) pour générer un classificateur de qualité d'image formé (74) pour prédire une évaluation de qualité d'image pour une image médicale d'entrée (44);
dans lequel la boîte de dialogue utilisateur d'évaluation de la qualité d'image (70) fournit des évaluations de qualité d'image contraintes pour la sélection de l'utilisateur comprenant au moins une évaluation de bonne qualité d'image et une évaluation de qualité d'image médiocre;
dans lequel, pendant une session de lecture d'images médicales, l'affichage des évaluations de qualité attribuées aux images médicales de session (44) comprend l'affichage que toute image médicale de session attribuée à l'évaluation de mauvaise qualité d'image doit être réacquise;
dans lequel l'apprentissage automatique comprend l'exécution d'un apprentissage en profondeur comprenant l'apprentissage d'un réseau neuronal (58) qui extrait des caractéristiques d'image en tant que sorties d'une ou plusieurs couches neuronales du réseau neuronal; dans lequel l'apprentissage en profondeur utilise en outre des métadonnées sur les images en tant qu'entrées dans le réseau neuronal (58), les métadonnées sur les images comprenant un ou plusieurs parmi:
la modalité d'image, la raison de l'examen et les antécédents du patient stockés dans les archives (54); et
dans lequel le dispositif d'imagerie médicale est l'un quelconque des éléments suivants: un appareil à rayons X, un appareil à résonance magnétique, un appareil de tomographie par ordinateur, un appareil à ultrasons, un appareil de tomographie par émission de positrons, un appareil de tomographie par émission à photon unique, et des combinaisons de ceux-ci.

6. Support lisible par ordinateur non transitoire selon la revendication 5, dans lequel, au cours d'une session de lecture d'images médicales, des notes de qualité sont attribuées aux images médicales de session (44) en entrant les images médicales de session dans le classificateur de qualité d'image formé (74).

7. Support lisible par ordinateur non transitoire selon l'une quelconque des revendications 5 et 6, dans lequel le procédé comprend en outre:
afficher la boîte de dialogue utilisateur d'évaluation de la qualité d'image (70) tout en affichant des images médicales stockées dans l'archive (20, 54); et
recevoir, via le dialogue utilisateur d'évaluation d'image (70), au moins l'une des évaluations de qualité d'image audio ou visuelle pour les images médicales stockées dans les archives (20, 54) et affichées sur le poste de travail médical (10).

8. Support lisible par ordinateur non transitoire selon la revendication 5, 6 ou 7, dans lequel l'apprentissage en profondeur n'opère pas sur des caractéristiques d'image identifiées manuellement.

9. Support lisible par ordinateur non transitoire selon l'une quelconque des revendications 5 à 8, dans lequel le procédé comprend en outre:
mettre à jour l'apprentissage automatique pour mettre à jour le classificateur de qualité d'image formé (74) lorsque des images médicales supplémentaires sont stockées dans l'archive (20, 54) ayant reçu des évaluations de qualité d'image via le dialogue utilisateur d'évaluation de qualité d'image.
